# EUROPEAN PATENT APPLICATION

(11) **EP 4 714 540 A1**
(43) Date of publication of application: **25.03.2026**
(21) Application number: 24806551.8
(22) Date of filing: 13.05.2024
(51) Int. Cl.: B01J 23/00, B01J 29/76, C07C 29/50, C07C 31/04

(54) **BIFUNCTIONAL CATALYST FOR PREPARING METHANOL BY MEANS OF OXIDATION OF METHANE COUPLED WITH HYDROGENATION OF CARBON DIOXIDE, AND PREPARATION METHOD THEREFOR AND USE THEREOF**

(30) Priority: 15.05.2023 CN 202310544092
(71) Applicant: East China Normal University, Shanghai 200062 (CN)
(72) Inventor: HUANG, Aisheng, Shanghai 200062 (CN); LI, Yanhong, Shanghai 200062 (CN)
(74) Representative: Mewburn Ellis LLP
(86) International application number: PCT/CN2024/092880
(87) International publication number: WO 2024/235200

(57) **Abstract**

Disclosed in the present invention are a bifunctional catalyst for preparing methanol by means of oxidation of methane coupled with hydrogenation of carbon dioxide, and a preparation method therefor and the use thereof. Specifically, the bifunctional catalyst comprises a first catalyst for catalyzing the oxidation of methane to prepare methanol and a second catalyst for catalyzing the hydrogenation of carbon dioxide to prepare methanol. The bifunctional catalyst can prepare methanol with a high conversion rate of methane and high selectivity of methanol when there is no exogenous oxygen source, which further consumes carbon dioxide during the reaction process, and has a high industrial application value.

## Description

### Technical field

The present invention relates to the catalytic synthesis field, specifically, the present invention relates to bifunctional catalyst for preparing methanol by means of oxidation of methane coupled with hydrogenation of carbon dioxide, and preparation method therefor and use thereof.

### Background

Methane is the main component of natural gas, with abundant reserves and low prices, making it a promising energy and chemical resource. In recent years, methane has received increasing attention as a clean fossil energy and chemical raw material. However, most natural gas production area is far from where it is used, and methane is difficult to liquefy at room temperature, making it expensive to transport over long distances. Therefore, converting methane into chemicals or liquid fuels such as methanol, which are easy to store and transport, will greatly reduce transportation costs and is of great significance to the conversion and utilization of methane. However, due to the high stability and weak polarity of the carbon-hydrogen bonds in methane molecule, the conversion of methane to methanol is extremely challenging and usually requires harsh reaction conditions such as high temperature and high pressure. However, due to the significantly lower bond energy of carbon hydrogen bonds in methanol molecules compared to methane molecules, methane is easily over-oxidized to produce carbon dioxide under high temperature and pressure conditions after activation. Therefore, methane conversion rate and methanol selectivity have always been considered as an irreconcilable contradiction. At present, the industrial methane-to-methanol conversion process usually adopts an indirect approach, where methane is firstly converted to syngas by steam reforming, and then the syngas is catalytically converted to methanol. Due to the energy intensive nature of methane steam reforming, the high energy consumption and cost of production greatly constrains the conversion and utilization of methane. Therefore, exploring and developing technologies and pathways for directly converting methane to methanol under mild conditions is of great significance.

In the past few decades, efforts have been made both domestically and internationally to explore effective catalysts or reaction strategies to promote methane activation while suppressing excessive oxidation of methanol. At present, there are two main pathways for catalytic oxidation of methane: liquid-phase oxidation and gas-phase oxidation. The reaction conditions of liquid-phase catalytic oxidation are relatively mild and can achieve high methanol selectivity, but usually require the use of noble metal catalysts as well as expensive oxidants or highly corrosive reaction media to break the carbon hydrogen bonds of methane molecules, resulting in high production costs, serious pollution, and inability to achieve large-scale industrial applications. In addition, the separation and recovery of methanol in liquid-phase oxidation reactions is also a major obstacle for large-scale industrial applications. Compared with liquid-phase catalytic oxidation process, gas-phase catalytic oxidation process is more suitable for downstream industrial applications of methanol due to easier recovery of product and catalyst. Inspired by the catalytic system of methane monooxygenase, zeolite molecular sieves exchanged with metal ions such as Cu, Fe, and Co can gradually convert methane to methanol in a cyclic reaction process under the synergistic action of different oxidants such as NOx, H₂O₂, O₂, and H₂O. Among them, the most interesting is that Sushkevich et al. proposed using water vapor as a single mild oxidant to selectively convert methane to methanol under the catalysis of Cu-MOR molecular sieve (Sushkevich et al., Science, 2017, 356, 523-5275). However, in order to achieve the goal of methanol production by cyclic oxidation of methane, it is necessary to constantly switch reaction temperatures and intermittently feed to activate oxidants and desorb the methanol products, making this reaction pathway still unable to meet the requirements of industrial applications. It is obvious that continuous reaction processes are conducive to achieving higher spatiotemporal yield. Recently, Sun et al. (Chem, 2021, 71, 557-1568) reported that selective catalytic oxidation of methane can be achieved on Cu-CHA zeolite molecular sieves in the presence of water vapor and oxygen. However, the reaction still requires continuous introduction of water and oxygen, which is quite inconvenient for large-scale industrial applications. Therefore, there is still an urgent need to develop a continuous, simple, and effective catalyst and reaction strategy for direct catalytic oxidation of methane to methanol.

Based on the reaction process enhancement strategy, the catalytic oxidation of methane to produce methanol and the catalytic hydrogenation of carbon dioxide to produce methanol are coupled in one reactor. On the one hand, the by-product water generated by the hydrogenation of carbon dioxide can be continuously provided in situ to the catalytic oxidation of methane to produce methanol, serving as an oxidant for the catalytic oxidation of methane to produce methanol, which not only eliminates the need for the process of transporting water from the outside, but also exhausts the by-product water generated by the catalytic hydrogenation of carbon dioxide, thereby driving the hydrogenation reaction of carbon dioxide to produce methanol. On the other hand, for catalytic oxidation of methane to produce methanol directly, catalytic hydrogenation of carbon dioxide to produce methanol can quickly and continuously consume the reaction byproduct hydrogen, which helps to break through the thermodynamic equilibrium limitation of the reaction of catalytic oxidation of methane to produce methanol and thereby improving methane conversion rate. Therefore, under mild conditions, by coupling the two reactions of methane oxidation to produce methanol and carbon dioxide catalytic hydrogenation to produce methanol in one reactor through a bifunctional catalyst, it will effectively promote the continuous catalytic oxidation of methane to methanol, thus developing a novel, simple, and continuous pathway for direct production of methanol through methane catalytic oxidation, which is beneficial for the conversion and utilization of greenhouse gases CH₄ and CO₂.

### Summary of the invention

One purpose of the present invention is to provide a bifunctional catalyst for preparing methanol by means of oxidation of methane coupled with catalytic hydrogenation of carbon dioxide with a high methane conversion rate and the preparation method therefor.

In the first aspect of the present invention, provided is a bifunctional catalyst, comprising a first catalyst for catalyzing methane oxidation to prepare methanol and a second catalyst for catalyzing carbon dioxide hydrogenation to prepare methanol; wherein the first catalyst has a polydopamine-modified layer,
wherein, the weight ratio of the first catalyst to the second catalyst is 3:1 to 1:1, preferably 1.5:1 to 1:1; most preferably 1:1.

In another preferred embodiment, the first catalyst is a polydopamine-modified metal ion-exchanged zeolite molecular sieve, preferably, the metal ion-exchanged zeolite molecular sieve is selected from the group consisting of: iron ion-exchanged zeolite molecular sieve, copper ion-exchanged zeolite molecular sieve, cobalt ion-exchanged zeolite molecular sieve, nickel ion exchanged zeolite molecular sieve, or combinations thereof.

In another preferred embodiment, the second catalyst is selected from the group consisting of: metal oxide, metal alloy, noble metal, or combinations thereof.

In another preferred embodiment, the metal oxide is selected from the group consisting of: copper oxide, zinc oxide, aluminum oxide, zirconium oxide, or combinations thereof.

In another preferred embodiment, the metal alloy is selected from the group consisting of: InZr, CuZn, PdZn, CuAg, or combinations thereof.

In another preferred embodiment, the noble metal is selected from the group consisting of: Pd, Au, Pt, or combinations thereof.

In another preferred embodiment, the bifunctional catalyst is selected from the group consisting of: CZAZ@Cu-ZSM-5, CZAZ@Cu-MOR, CZAZ@Cu-CHA, CZAZ@Cu-BEA, CZAZ@Cu-FER, CZAZ/Cu-ZSM-5, CZAZ/Cu-MOR, CZAZ/Cu-CHA, CuO-ZnO@Cu-MOR, CuO-ZnO/Cu-MOR, CuO-ZnO-ZrO₂@Cu-MOR, CuO-ZnO-ZrO₂/Cu-MOR, CuO-ZnO-Al₂O₃@Cu-MOR, CuO-ZnO-ZrO₂/Cu-MOR, CZAZ/Fe-ZSM-5, CZAZ/Fe-MOR, or combinations thereof.

In the second aspect of the present invention, provided is a use of the bifunctional catalyst according to the first aspect of the present invention, wherein the bifunctional catalyst is used for preparing methanol by catalyzing methane oxidation coupled with carbon dioxide hydrogenation, wherein the bifunctional catalyst comprises a first catalyst for catalyzing methane oxidation to prepare methanol and a second catalyst for catalyzing carbon dioxide hydrogenation to prepare methanol.

In another preferred embodiment, the mass ratio of the first catalyst to the second catalyst is 3:1 to 1:1; preferably 15:1 to 1:1; most preferably 1:1.

In another preferred embodiment, the first catalyst is a polydopamine-modified metal ion-exchanged zeolite molecular sieve, preferably, the metal ion-exchanged zeolite molecular sieve is selected from the group consisting of: iron ion-exchanged zeolite molecular sieve, copper ion-exchanged zeolite molecular sieve, cobalt ion-exchanged zeolite molecular sieve, nickel ion exchanged zeolite molecular sieve, or combinations thereof.

In another preferred embodiment, the second catalyst is selected from the group consisting of: metal oxide, metal alloy, noble metal, or combinations thereof.

In another preferred embodiment, the metal oxide is selected from the group consisting of: copper oxide, zinc oxide, aluminum oxide, zirconium oxide, or combinations thereof.

In another preferred embodiment, the metal alloy is selected from the group consisting of: InZr, CuZn, PdZn, CuAg, or combinations thereof.

In another preferred embodiment, the noble metal is selected from the group consisting of: Pd, Au, Pt, or combinations thereof.

In another preferred embodiment, the bifunctional catalyst is selected from the group consisting of: CZAZ@Cu-ZSM-5, CZAZ@Cu-MOR, CZAZ@Cu-CHA, CZAZ@Cu-BEA, CZAZ@Cu-FER, CZAZ/Cu-ZSM-5, CZAZ/Cu-MOR, CZAZ/Cu-CHA, CuO-ZnO@Cu-MOR, CuO-ZnO/Cu-MOR, CuO-ZnO-ZrO₂@Cu-MOR, CuO-ZnO-ZrO₂/Cu-MOR, CuO-ZnO-Al₂O₃@Cu-MOR, CuO-ZnO-ZrO₂/Cu-MOR, CZAZ/Fe-ZSM-5, CZAZ/Fe-MOR, or combinations thereof.

In the third aspect of the present invention, provided is a method for preparing the bifunctional catalyst as described in the first aspect of the present invention, comprising the steps of: preparing the first catalyst and the second catalyst through a wet process or a dry process to obtain the bifunctional catalyst.

In another preferred embodiment, when preparing the bifunctional catalyst using a wet process, the method comprises the following steps:
(i) dissolving and dispersing a first catalyst precursor in C1-C6 alcoholic solvent to obtain a mixture;
(ii) adding the second catalyst to the mixture and reacting at room temperature to obtain the bifunctional catalyst.

In another preferred embodiment, the method further comprises: drying and calcining at a high temperature to obtain the bifunctional catalyst.

In another preferred embodiment, the first catalyst precursor is the corresponding hydroxide complex of the metal in the first catalyst, preferably Cu(OH)₂-Zn(OH)₂-Al(OH)₃-Zr(OH)₂.

In another preferred embodiment, the reaction temperature is 5-35 °C (room temperature); preferably 15-25 °C.

In another preferred embodiment, the reaction temperature is 18-30 h; preferably, 18-25 h.

In another preferred embodiment, the C1-C6 alcoholic solvent is ethanol.

In another preferred embodiment, the drying refers to drying at 100-150 °C; preferably 110-120 °C.

In another preferred embodiment, the high temperature is 300-500 °C; preferably 350-400 °C.

In another preferred embodiment, the calcination time is 2-8 h; preferably 3-4 h.

In another preferred embodiment, when preparing the bifunctional catalyst using a dry process, the method comprises the following steps:
mixing the dry first catalyst and second catalyst evenly to obtain the bifunctional catalyst;
wherein, the mass ratio of the first catalyst to the second catalyst is 3:1 to 1:1, preferably 1.5:1 to 1:1.

In the fourth aspect of the present invention, provided is a method for preparing methanol by catalyzing methane oxidation coupled with carbon dioxide hydrogenation, comprising the following steps:
in the same reactor, in the presence of the bifunctional catalyst as described in the first aspect of the present invention, under a condition of 100-400 °C, introducing a reaction inlet gas containing methane, carbon dioxide, and hydrogen to conduct the reaction, thereby producing methanol, wherein, the inlet gas space velocity of the reactor is 3000 to 10000 h⁻¹, and the inlet gas of the reaction contains 5 to 30% by volume of methane, 5 to 30% by volume of carbon dioxide, and 10 to 60% by volume of hydrogen.

In another preferred embodiment, in the reaction inlet gas, the volume content of methane is 18 to 30%, the volume content of carbon dioxide is 18 to 30%, and the volume content of hydrogen is 40 to 60%.

In another preferred embodiment, in the reaction inlet gas, the volume content of methane is 25%, the volume content of carbon dioxide is 25%, and the volume content of hydrogen is 50%.

In another preferred embodiment, the inlet gas space velocity of the reactor is 4000-8000 h⁻¹, preferably the inlet gas space velocity of the reactor is 5000 h⁻¹.

In another preferred embodiment, the reaction pressure is 1.5 to 4.0 MPa.

In another preferred embodiment, the reaction temperature is 200 to 300 °C.

It should be understood that within the scope of the present invention, the above-mentioned technical features of the present invention and the specific technical features described in the following embodiments can be combined with each other to form a new or preferred technical solution. Due to space limitations, it will not be repeated here.

### Description of the drawings

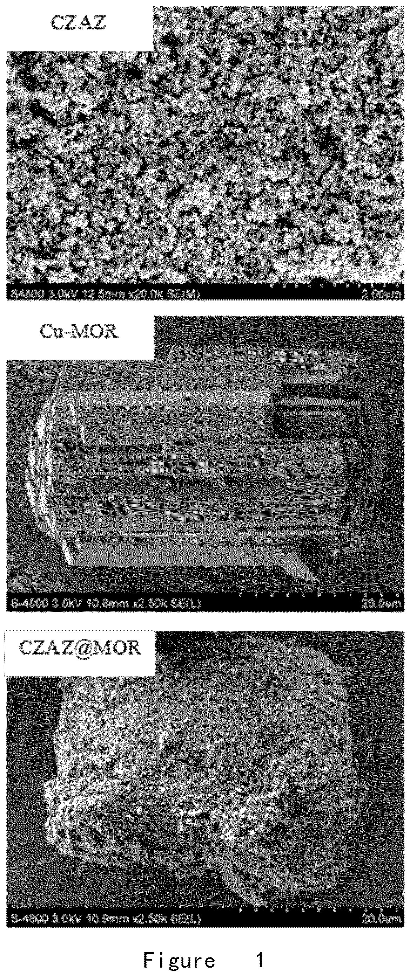

Figure 1 shows the scanning electron micrograph of CZAZ, Cu-MOR molecular sieve, and CZAZ@Cu-MOR bifunctional composite catalysts.

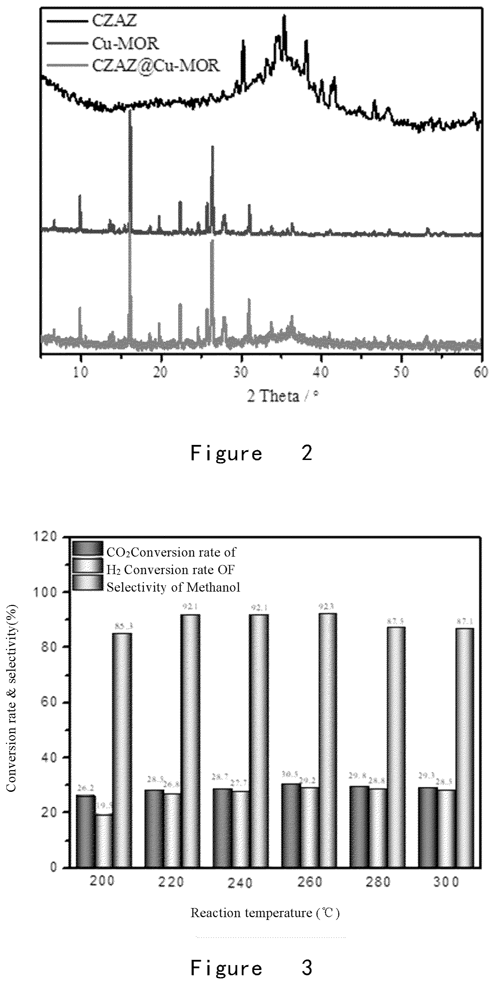

Figure 2 shows the X-ray diffraction spectrum of CZAZ, Cu-MOR molecular sieve, and CZAZ@Cu-MOR bifunctional composite catalysts.
Figure 3 shows the schematic diagram of catalytic performance of CZAZ@Cu-MOR bifunctional composite catalyst for producing methanol via methane catalytic oxidation coupled with carbon dioxide hydrogenation as a function of temperature.

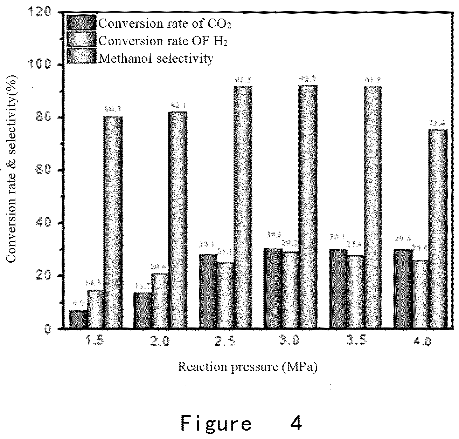

Figure 4 shows the schematic diagram of catalytic performance of CZAZ@Cu-MOR bifunctional composite catalyst for producing methanol via methane catalytic oxidation coupled with carbon dioxide hydrogenation as a function of pressure.

### Detailed Description of the Invention

After extensive and in-depth research and numerous experiments, the inventors have developed a bifunctional catalyst for methane oxidation coupled with carbon dioxide catalytic hydrogenation to prepare methanol with high methane conversion rate and high methanol selectivity. The bifunctional catalyst consists of a catalyst for methane oxidation to prepare methanol and a catalyst for carbon dioxide catalytic hydrogenation to prepare methanol, wherein the catalyst for carbon dioxide catalytic hydrogenation to prepare methanol is one or more of metal oxides, metal alloys, and noble metals; the catalyst for methane oxidation to prepare methanol is a molecular sieve exchanged with melt ions such as iron, copper, and cobalt ions. The bifunctional catalyst can provide methane and water, byproducts of methanol production from carbon dioxide hydrogenation, in situ for methane catalytic oxidation to prepare methanol, thereby greatly increasing the selectivity of methanol and the conversion rate of carbon dioxide. On this basis, the inventors completed the present invention.

### Term

As used herein, the term "room temperature" refers to 5 to 30 °C; preferably 20 to 30 °C.

### Polydopamine-modified metal ion-exchanged molecular sieve

The first catalyst of the present invention is a metal ion-exchanged molecular sieve with polydopamine modification. It can be prepared using common hydrothermal synthesis and ion exchange methods in this field. Typically, comprising the following steps:
Dissolving dopamine hydrochloride, copper sulfate, hydrogen peroxide, and tris (hydroxymethyl) aminomethane in distilled water, stirring well, and then adding zeolite molecular sieves exchanged with metal ion such as iron, copper, cobalt, and nickel. The molecular sieves include ZSM-5, MOR, CHA, BEA, FER, etc. The reaction is carried out at 20 to 35 °C for 2 to 4 h to support a layer of polydopamine on the surface of the metal (such as iron, copper, cobalt, and nickel) ion-exchanged zeolite molecular sieves in order to promote the supporting of active component for the preparation of methanol by catalytic hydrogenation of carbon dioxide on the surface of the molecular sieve. Subsequently, centrifuging and washing the zeolite molecular sieve with distilled water 3 times, followed by drying at 30 °C to obtain polydopamine modified metal (such as iron, copper, cobalt, and nickel) ion-exchanged zeolite molecular sieves.

### Bifunctional catalyst for prepare methanol by means of oxidation of methane coupled with hydrogenation of carbon dioxide

The present invention provides a bifunctional catalyst with a high catalytic efficiency, high methanol selectivity, and high methane conversion rate. The bifunctional catalyst comprises a first catalyst and a second catalyst, wherein the first catalyst is used for catalyzing methane oxidation to prepare methanol, and the second catalyst is used for catalyzing carbon dioxide hydrogenation to prepare methanol; the mass ratio of the first catalyst to the second catalyst is 3:1 to 1:1; preferably 1.5:1 to 1:1; most preferably 1:1.

In one embodiment, the first catalyst is a polydopamine-modified metal ion-exchanged zeolite molecular sieve. Preferably, the metal ion-exchanged zeolite molecular sieve is selected from the group consisting of: iron ion-exchanged zeolite molecular sieve, copper ion-exchanged zeolite molecular sieve, cobalt ion-exchanged zeolite molecular sieve, nickel ion exchanged zeolite molecular sieve, or combinations thereof.

Preferably, the first catalyst is a metal ion-exchanged zeolite molecular sieve, preferably, the metal ion-exchanged zeolite molecular sieve is selected from the group consisting of: iron ion-exchanged zeolite molecular sieve, copper ion-exchanged zeolite molecular sieve, cobalt ion-exchanged zeolite molecular sieve, or combinations thereof; more preferably Cu-MOR molecular sieve.

Preferably, the zeolite molecular sieve is selected from the group consisting of: ZSM-5 zeolite molecular sieve, MOR zeolite molecular sieve, CHA zeolite molecular sieve, or combinations thereof.

Preferably, the iron ion-exchanged zeolite molecular sieve is Fe-ZSM-5 molecular sieve.

Preferably, the copper ion-exchanged zeolite molecular sieve is Cu-ZSM-5 molecular sieve, Cu-MOR molecular sieve, Cu-CHA molecular sieve, or combinations thereof.

Preferably, the first catalyst is a polydopamine-modified molecular sieve selected from the group consisting of: Cu-MOR, Cu-CHA, Cu-BEA, Cu-ZSM-5, Cu FER, Fe-MOR, Fe-ZSM-5, or combinations thereof.

In one embodiment, the second catalyst is selected from the group consisting of: metal oxide, metal alloy, noble metal, or combinations thereof, wherein the metal oxide is selected from the group consisting of: copper oxide, zinc oxide, aluminum oxide, zirconium oxide, or combinations thereof; the metal alloy is selected from the group consisting of: InZr, CuZn, PdZn, CuAg, or combinations thereof; the noble metal is selected from the group consisting of: Pd, Au, Pt, or combinations thereof.

Preferably, the second catalyst is selected from the group consisting of: CuO-ZnO-Al₂O₃-ZrO₂ (CZAZ), CuO-ZnO, CuO-ZnO-ZrO₂, CuO-ZnO-Al₂O₃, or combinations thereof.

Preferably, the bifunctional catalyst is selected from the group consisting of: CZAZ@Cu-ZSM-5, CZAZ@Cu-MOR, CZAZ@Cu-CHA, CZAZ@Cu-BEA, CZAZ@Cu-FER, CZAZ/Cu-ZSM-5, CZAZ/Cu-MOR, CZAZ/Cu-CHA, CuO-ZnO@Cu-MOR, CuO-ZnO/Cu-MOR, CuO-ZnO-ZrO₂@Cu-MOR, CuO-ZnO-ZrO₂/Cu-MOR, CuO-ZnO-Al₂O₃@Cu-MOR, CuO-ZnO-ZrO₂/Cu-MOR, CZAZ/Fe-ZSM-5, CZAZ/Fe-MOR, or combinations thereof.

More preferably, the bifunctional catalyst is selected from the group consisting of: CZAZ@Cu-ZSM-5, CZAZ@Cu-MOR, CZAZ@Cu-CHA, CZAZ/Cu-MOR, CZAZ/Cu-CHA, CuO-ZnO-ZrO₂@Cu-MOR, CuO-ZnO-Al₂O₃@Cu-MOR, CuO-ZnO-ZrO₂/Cu-MOR, CuO-ZnO-ZrO₂/Cu-MOR, or combinations thereof.

### Preparation method of the bifunctional catalyst

### Method 1: preparation of the bifunctional catalyst by impregnation or precipitation-deposition method:

1) The active components for catalytic hydrogenation of carbon dioxide to prepare methanol prepared by co-precipitation method include CuO-ZnO, CuO-ZrO₂, CuO-ZnO-Al₂O₃, CuO-ZnO-ZrO₂, CuO-ZnO-Al₂O₃-ZrO₂, etc. The active components for catalytic hydrogenation of carbon dioxide to prepare methanol are dried or dissolved and dispersed in ethanol for later use.
2) Dissolving dopamine hydrochloride, copper sulfate, hydrogen peroxide, and tris (hydroxymethyl) aminomethane in distilled water, stirring well, and then adding metal (such as iron, copper, cobalt, and nickel) ion-exchanged zeolite molecular sieves. The molecular sieves include ZSM-5, MOR, CHA, BEA, FER, etc. The reaction is carried out at 20-35 °C for 2-4 h to load a layer of polydopamine on the surface of the metal (such as iron, copper, cobalt, and nickel) ion-exchanged zeolite molecular sieves, in order to promote the loading of active components for catalytic hydrogenation of carbon dioxide to prepare methanol on the surface of the molecular sieve. Subsequently, centrifuging and washing the zeolite molecular sieve with distilled water 3 times, followed by drying at 30 °C to obtain polydopamine modified metal (such as iron, copper, cobalt, and nickel) ion-exchanged zeolite molecular sieves.
3) Adding zeolite molecular sieves modified with polydopamine and exchanged with metal ions such as iron, copper, cobalt, and nickel to ethanol containing the catalyst precursors for the catalytic hydrogenation of carbon dioxide to prepare methanol and reacting at 25 °C for 2 hours to obtain the bifunctional catalyst for producing methanol via methane catalytic oxidation coupled with carbon dioxide hydrogenation.

### Method 2: preparation of the bifunctional catalyst by physical mixing:

Mixing the second catalyst for catalytic hydrogenation of carbon dioxide to prepare methanol with zeolite molecular sieves modified with polydopamine and exchanged with metal ions such as iron, copper, cobalt, and nickel (first catalyst) in proportion to obtain the bifunctional catalyst for producing methanol via methane catalytic oxidation coupled with carbon dioxide hydrogenation; wherein, the mass ratio of the first catalyst to the second catalyst is 3:1 to 1:1; more preferably 1.5:1 to 1:1; most preferably 1:1.

### Performance evaluation of the bifunctional catalyst for catalyzing methane oxidation coupled with carbon dioxide hydrogenation to prepare methanol

The catalytic performance of the bifunctional catalyst for methane catalytic oxidation coupled with carbon dioxide hydrogenation to prepare methanol is evaluated in a conventional fixed-bed reactor. The prepared bifunctional catalysts are placed in a same reactor. H₂, CO₂, and CH₄ are mixed thoroughly in a gas mixing device, and then transferred to the reactor. The flow rate of each gas is controlled by a mass flow controller. The reactions are carried out under conditions of pressure ranging from 1.5 to 4.0 MPa, temperature ranging from 100 to 400 °C, and gas hourly space velocity (GHSV) ranging from 3000 to 10000 h⁻¹. The volume percentages of each gas are 5 to 30% by volume of methane, 5 to 30% by volume of carbon dioxide, and 10 to 60% by volume of hydrogen, respectively. The reaction products and exhaust gases are analyzed by a gas chromatography (Agilent 7890B) equipped with TCD and FID detectors, and the conversion rates of CH₄ and CO₂ and the selectivity of methanol are calculated.

In a preferred embodiment, the volume percentages of each gas are 18 to 30% by volume of methane, 18 to 30% by volume of carbon dioxide, and 40 to 60% by volume of hydrogen, respectively.

In a preferred embodiment, the gas hourly space velocity (GHSV) is 5000 h⁻¹.

**The main advantages of the present invention compared with prior art include:**
(a) The byproduct water of hydrogenation of carbon dioxide catalyzed by the second catalyst in the bifunctional catalyst of the present application, can be continuously provided in situ to catalytic oxidation of methane to prepare methanol to serve as an oxidant for methane catalytic oxidation to prepare methanol, which not only eliminates the need of the process of transporting water from the outsideto, but also exhausts the byproduct water generated by catalytic hydrogenation of carbon dioxide, thereby driving the reaction of carbon dioxide hydrogenation to prepare methanol.
(b) For the direct production reaction of methanol by catalytic oxidation of methane, catalytic hydrogenation of carbon dioxide to prepare methanol can quickly and continuously consume the reaction byproduct hydrogen, which helps to break through the thermodynamic equilibrium limitation of the reaction of methane catalytic oxidation to prepare methanol and improve methane conversion rate.
(c) The bifunctional catalyst of the present invention can be used to prepare methanol downstream of methane and/or carbon dioxide exhaust gas, which is environmentally friendly.

The present invention will be further explained below in conjunction with specific embodiments. It should be understood that these embodiments are only used to illustrate the present invention and not to limit the scope of the present invention. The experimental methods without specific conditions specified in the following examples are usually in accordance with conventional conditions or conditions recommended by the manufacturer. Unless otherwise specified, percentages and parts are calculated by weight.

### Example 1

### Step 1: Preparation of the active component CuO-ZnO-Al₂O₃-ZrO₂ (CZAZ) for catalytic hydrogenation of carbon dioxide to prepare methanol

14.49 g of copper nitrate trihydrate, 17.85 g of zinc nitrate hexahydrate, 22.5 g of aluminum nitrate nonahydrate, and 4.05 g of zirconium oxychloride octahydrate were dissolved in 50 g of distilled water. Then saturated sodium carbonate solution was added slowly and dropwise and the precursor of CuO-ZnO-Al₂O₃-ZrO₂ catalyst was prepared by co-precipitation method at 80 °C. Upon completion of the dropwise addition, the mixture continued to be stirred for 10 minutes, and then performed static aging for 2 hours. The obtained precipitate was washed thoroughly with distilled water, filtered and dried, and then calcined in a muffle furnace at 350 °C and under air atmosphere for 4 hours to obtain CuO-ZnO-Al₂O₃-ZrO₂, alternatively, the precipitate was filtered, dried and dissolved dispersedly in ethanol for later use.

### Step 2: Polydopamine modified copper ion-exchanged ZSM-5, MOR, CHA, BEA, FER molecular sieves

5 g of ZSM-5, MOR, CHA, BEA, FER molecular sieves were added to 250 ml of 0.05 mol/L copper nitrate solution. The mixture was stirred under 50 °C water bath for 24 hours, and then filtered. The resulting molecular sieves were rinsed with distilled water, and dried at 120 °C for 12 hours. The above steps were repeated twice to fully exchange with copper ions. The dried powders were calcined at 500 °C and under air atmosphere for 4 hours to obtain copper ion-exchanged Cu-ZSM-5, Cu-MOR, Cu-CHA, Cu-BEA, Cu-FER molecular sieves.

0.15 g of dopamine hydrochloride, 0.12 g of copper sulfate hexahydrate, and 0.39 g of hydrogen peroxide were dissolved in 150 ml of 10 mol/L tris (hydroxymethyl) aminomethane. The mixture was stirred well, and then added with 15 g of copper ion-exchanged zeolite molecular sieves such as Cu-ZSM-5, Cu-MOR, Cu-CHA, Cu-BEA, Cu-FER, etc., and reacted at 25 °C for 3 hours to load a layer of polydopamine on the surface of molecular sieves such as Cu-ZSM-5, Cu-MOR, Cu-CHA, Cu-BEA, Cu-FER. Subsequently, the molecular sieves were centrifuged and washed with distilled water three times, followed by drying at 30 °C to obtain polydopamine modified molecular sieves such as Cu-ZSM-5, Cu-MOR, Cu-CHA, Cu-BEA, Cu-FER, etc.

### Step 3: Preparation of bifunctional catalysts

3 g of polydopamine modified Cu-ZSM-5, Cu-MOR, Cu-CHA, Cu-BEA, Cu-FER molecular sieves were added to 10 ml of ethanol solution containing 3 g of CZAZ catalyst precursor. After reacting at 25 °C for 2 hours, the obtained molecular sieves were centrifuged and washed three times with distilled water, and then dried at 110 °C, and finally calcined in a muffle furnace at 350 °C and under air atmosphere for 4 hours to obtain the bifunctional catalysts such as CZAZ@Cu-ZSM-5, CZAZ@Cu-MOR, CZAZ@Cu-CHA, CZAZ@Cu-BEA, and CZAZ@Cu-FER for producing methanol via methane catalytic oxidation coupled with carbon dioxide hydrogenation.

### Step 4: Preparation of methanol by catalytic oxidation of methane coupled with hydrogenation of carbon dioxide

The prepared bifunctional catalysts such as CZAZ@Cu-ZSM-5, CZAZ@Cu-MOR, CZAZ@Cu-CHA, CZAZ@Cu-BEA, and CZAZ@Cu-FER were separately placed in catalytic reactors. H₂, CO₂, and CH₄ with a volume ratio of 2:1:1 were mixed thoroughly in a gas mixing device, and transferred to the reactor. The reaction was controlled to be carried out under the conditions of pressure ranging from 1.5 to 4.0 MPa, temperature ranging from 200 to 300 °C, and gas hourly space velocity (GHSV) of 5000 h⁻¹. The reaction products were analyzed by gas chromatography and the conversion rates of CH₄ and CO₂ and the selectivity of methanol were calculated.

### Example 2

### Step 1: Preparation of the active component CuO-ZnO-Al₂O₃-ZrO₂ (CZAZ) for catalytic hydrogenation of carbon dioxide to prepare methanol

The process is the same as step 1 in Example 1.

### Step 2: Copper ion-exchanged ZSM-5, MOR, CHA molecular sieves

The process is the same as Step 2 in Example 1.

### Step 3: Preparation of bifunctional catalysts

3 g of copper ion-exchanged Cu-ZSM-5, Cu-MOR, and Cu-CHA molecular sieves were mixed evenly with 3 g of CZAZ, respectively. Subsequently, the molecular sieves were calcined in a muffle furnace at 350 °C and under air atmosphere for 4 hours to obtain the bifunctional catalysts CZAZ/Cu-ZSM-5, CZAZ/Cu-MOR, and CZAZ/Cu-CHA with a weight ratio of 1:1 for producing methanol via methane catalytic oxidation coupled with carbon dioxide hydrogenation.

### Step 4: Preparation of methanol by catalytic oxidation of methane coupled with hydrogenation of carbon dioxide

The prepared bifunctional catalysts such as CZAZ/Cu-ZSM-5, CZAZ/Cu-MOR, and CZAZ/Cu-CHA were separately placed in catalytic reactors. The catalytic performance of the bifunctional catalysts was evaluated according to the method in Step 4 of Example 1.

### Example 3

### Step 1: Preparation of the active component CuO-ZnO for catalytic hydrogenation of carbon dioxide to prepare methanol

14.49 g of copper nitrate trihydrate and 17.85 g of zinc nitrate hexahydrate were dissolved in 50 g of distilled water. Then saturated sodium carbonate solution was added slowly and dropwise and the precursor of CuO-ZnO catalyst was prepared by co-precipitation method at 80 °C. Upon completion of the dropwise addition, the mixture continued to be stirred for 10 minutes, and then performed static aging for 2 hours. The obtained precipitate was washed thoroughly with distilled water, filtered and dried, and then calcined in a muffle furnace at 350 °C and under air atmosphere for 4 hours to obtain CuO-ZnO, or the precipitate was filtered, dried and dissolved dispersedly in ethanol for later use.

### Step 2: Polydopamine modified copper ion-exchanged Cu-MOR molecular sieve:

Cu-MOR molecular sieve was prepared using the same method as in step 2 of Example 1.

### Step 3: Preparation of bifunctional catalysts CuO-ZnO@Cu-MOR and CuO-ZnO/Cu-MOR

Bifunctional catalyst CuO-ZnO@Cu-MOR was prepared using the same method as in step 3 of Example 1.

Bifunctional catalyst CuO-ZnO/Cu-MOR with a weight ratio of 1:1 was prepared using the same method as in step 3 of Example 2.

### Step 4: Preparation of methanol by catalytic oxidation of methane coupled with hydrogenation of carbon dioxide

The prepared bifunctional catalysts such as CuO-ZnO@Cu-MOR and CuO-ZnO/Cu-MOR were separately placed in catalytic reactors. The catalytic performance of the bifunctional catalysts was evaluated according to the method in Step 4 of Example 1.

### Example 4

### Step 1: Preparation of the active component CuO-ZnO-ZrO₂ for catalytic hydrogenation of carbon dioxide to prepare methanol

14.49 g of copper nitrate trihydrate, 17.85 g of zinc nitrate hexahydrate, and 4.05 g of zirconium oxychloride octahydrate were dissolved in 50 g of distilled water. Then saturated sodium carbonate solution was added slowly and dropwise and the precursor of CuO-ZnO-ZrO₂ catalyst was prepared by co-precipitation method at 80 °C. Upon completion of the dropwise addition, the mixture continued to be stirred for 10 minutes, and then performed static aging for 2 hours. The obtained precipitate was washed thoroughly with distilled water, filtered and dried, and then calcined in a muffle furnace at 350 °C and under air atmosphere for 4 hours to obtain CuO-ZnO-ZrO₂, alternatively, the precipitate was filtered, dried and dissolved dispersedly in ethanol for later use.

### Step 2: Polydopamine modified copper ion-exchanged Cu-MOR molecular sieve:

Cu-MOR molecular sieve was prepared using the same method as in step 2 of Example 1.

### Step 3: Preparation of bifunctional catalysts CuO-ZnO-ZrO₂@Cu-MOR and CuO-ZnO-ZrO₂/Cu-MOR

Bifunctional catalyst CuO-ZnO-ZrO₂@Cu-MOR was prepared using the same method as in step 3 of Example 1.

Bifunctional catalyst CuO-ZnO-ZrO₂/Cu-MOR with a weight ratio of 1:1 was prepared using the same method as in step 3 of Example 2.

### Step 4: Preparation of methanol by catalytic oxidation of methane coupled with hydrogenation of carbon dioxide

The prepared bifunctional catalysts such as CuO-ZnO-ZrO₂@Cu-MOR and CuO-ZnO-ZrO₂/Cu-MOR were separately placed in catalytic reactors. The catalytic performance of the bifunctional catalysts was evaluated according to the method in Step 4 of Example 1.

### Example 5

### Step 1: Preparation of the active component CuO-ZnO-Al₂O₃ for catalytic hydrogenation of carbon dioxide to prepare methanol

14.49 g of copper nitrate trihydrate, 17.85 g of zinc nitrate hexahydrate, and 22.5 g of aluminum nitrate nonahydrate were dissolved in 50 g of distilled water. Then saturated sodium carbonate solution was added slowly and dropwise and the precursor of CuO-ZnO-Al₂O₃ catalyst was prepared by co-precipitation method at 80 °C. Upon completion of the dropwise addition, the mixture continued to be stirred for 10 minutes, and then performed static aging for 2 hours. The obtained precipitate was washed thoroughly with distilled water, filtered and dried, and then calcined in a muffle furnace at 350 °C and under air atmosphere for 4 hours to obtain CuO-ZnO-Al₂O₃, alternatively, the precipitate was dissolved dispersedly in ethanol for later use.

### Step 2: Polydopamine modified copper ion-exchanged Cu-MOR molecular sieve:

Cu-MOR molecular sieve was prepared using the same method as in step 2 of Example 1.

### Step 3: Preparation of bifunctional catalysts CuO-ZnO-Al₂O₃@Cu-MOR and CuO-ZnO-Al₂O₃/Cu-MOR

Bifunctional catalyst CuO-ZnO-Al₂O₃@Cu-MOR was prepared using the same method as in step 3 of Example 1.

Bifunctional catalyst CuO-ZnO-Al₂O₃/Cu-MOR with a weight ratio of 1:1 was prepared using the same method as in step 3 of Example 2.

### Step 4: Preparation of methanol by catalytic oxidation of methane coupled with hydrogenation of carbon dioxide

The prepared bifunctional catalysts such as CuO-ZnO-Al₂O₃@Cu-MOR and CuO-ZnO-Al₂O₃/Cu-MOR were separately placed in catalytic reactors. The catalytic performance of the bifunctional catalysts was evaluated according to the method in Step 4 of Example 1.

### Example 6

### Step 1: Preparation of the active component CuO-ZnO-Al₂O₃-ZrO₂ (CZAZ) for catalytic hydrogenation of carbon dioxide to prepare methanol

CZAZ was prepared using the same method as in Step 1 of Example 1.

### Step 2: Preparation of iron ion-exchanged Fe-ZSM-5 molecular sieve:

5 g of ZSM-5 molecular sieves were added to 250 ml of 0.05 M iron nitrate solution. The mixture was stirred under 50 °C water bath for 24 hours, and then filtered. The resulting molecular sieves were rinsed with distilled water, and dried at 120 °C for 12 hours. The above steps were repeated twice to fully exchange with iron ions. The dried molecular sieve powder was calcined at 500 °C and under air atmosphere for 4 hours to obtain iron ion-exchanged Fe-ZSM-5 molecular sieve.

### Step 3: Preparation of bifunctional catalyst CZAZ/Fe-ZSM-5

Fe-ZSM-5 molecular sieve and CZAZ of equal mass were mixed uniformly to prepare the bifunctional catalyst CZAZ/Fe-ZSM-5.

### Step 4: Preparation of methanol by catalytic oxidation of methane coupled with hydrogenation of carbon dioxide

The prepared bifunctional catalyst CZAZ/Fe-ZSM-5 was placed in catalytic reactor. The catalytic performance of the bifunctional catalyst was evaluated according to the method in Step 4 of Example 1.

### Example 7

### Step 1: Preparation of the active component CuO-ZnO-Al₂O₃-ZrO₂ (CZAZ) for catalytic hydrogenation of carbon dioxide to prepare methanol:

CZAZ was prepared using the same method as in Step 1 of Example 1.

### Step 2: Preparation of iron ion-exchanged Fe-MOR molecular sieve:

5 g of MOR molecular sieves were added to 250 ml of 0.05 M iron nitrate solution. The mixture was stirred under 50 °C water bath for 24 hours, and then filtered. The resulting molecular sieves were rinsed with distilled water, and dried at 120 °C for 24 hours. The above steps were repeated twice to fully exchange with iron ions. The dried molecular sieve powder was calcined at 500 °C and under air atmosphere for 4 hours to obtain iron ion-exchanged Fe-MOR molecular sieve.

### Step 3: Preparation of bifunctional catalyst

Fe-ZSM-5 molecular sieve and CZAZ of equal mass were mixed uniformly to prepare the bifunctional catalyst CZAZ/Fe-MOR.

### Step 4: Preparation of methanol by catalytic oxidation of methane coupled with hydrogenation of carbon dioxide

The prepared bifunctional catalyst CZAZ/Fe-MOR was placed in a catalytic reactor. The catalytic performance of the bifunctional catalyst was evaluated according to the method in Step 4 of Example 1.

### Example 8 (Comparative Example)

### Step 1: Preparation of the active component CuO-ZnO-Al₂O₃-ZrO₂ for catalytic hydrogenation of carbon dioxide to prepare methanol:

CuO-ZnO-Al₂O₃-ZrO₂ was prepared using the same method as in step 1 of Example 1.

### Step 2: Preparation of copper ion-exchanged Cu-MOR molecular sieve (without dopamine modification):

5 g of MOR molecular sieves were added to 250 ml of 0.05 mo/L copper nitrate solution. The mixture was stirred under 50 °C water bath for 24 hours, and then filtered. The resulting molecular sieves were rinsed with distilled water, and dried at 120 °C for 24 hours. The above steps were repeated twice to fully exchange with copper ions. The dried molecular sieve powder was calcined at 500 °C and under air atmosphere for 4 hours to obtain copper ion-exchanged Cu-MOR molecular sieve.

### Step 3: Preparation of bifunctional catalyst

3 g of copper ion-exchanged Cu-MOR molecular sieves were added to 10 ml of ethanol solution containing 3 g of CZAZ catalyst precursor, and reacted at 25 °C for 2 hours. The obtained molecular sieves were centrifuged and washed with distilled water three times, and then dried at 110 °C. Finally, the obtained molecular sieves were calcined in a muffle furnace at 350 °C and under air atmosphere for 4 hours to obtain the bifunctional catalyst CZAZ@Cu-MOR for producing methanol via methane catalytic oxidation coupled with carbon dioxide hydrogenation.

### Step 4: Preparation of methanol by catalytic oxidation of methane coupled with hydrogenation of carbon dioxide

The prepared bifunctional catalyst CZAZ@Cu-MOR molecular sieve was placed in a catalytic reactor. The catalytic performance of the bifunctional catalyst was evaluated according to the method in Step 4 of Example 1.

### Example 9 (Comparative Example)

### Step 1: Preparation of the active component CuO-ZnO-Al₂O₃-ZrO₂ for catalytic hydrogenation of carbon dioxide to prepare methanol:

CuO-ZnO-Al₂O₃-ZrO₂ was prepared using the same method as in step 1 of Example 1.

### Step 2: Preparation of copper ion-exchanged Cu-MOR molecular sieve

Cu-MOR molecular sieve was prepared using the same method as in step 3 of Example 1.

### Step 3: Preparation of methanol by catalytic oxidation of methane coupled with hydrogenation of carbon dioxide

The prepared bifunctional catalysts CuO-ZnO-Al₂O₃-ZrO₂ and Cu-MOR molecular sieve was placed in catalytic reactor, respectively. The catalytic performance of the bifunctional catalyst was evaluated according to the method in Step 4 of Example 1.

The following table shows the catalytic performance at 260 °C and 3MPa of the catalysts prepared in various examples of the present invention

| **Examples** | **catalyst** | **Conversion rate of CH₄ (%)** | **Conversion rate of CO₂ (%)** | **Methanol selectivity (%)** |
|---|---|---|---|---|
| 1 | CZAZ@Cu-ZSM-5 | 22.5 | 19.7 | 80.6 |
| | CZAZ@Cu-MOR | 29.2 | 30.5 | 92.3 |
| | CZAZ@Cu-CHA | 24.3 | 21.2 | 84.9 |
| | CZAZ@Cu-BEA | 18.9 | 17.5 | 69.3 |
| | CZAZ@Cu-FER | 23.1 | 15.4 | 46.4 |
| 2 | CZAZ/Cu-ZSM-5 | 20.1 | 18.8 | 78.3 |
| | CZAZ/Cu-MOR | 28.2 | 29.7 | 90.7 |
| | CZAZ/Cu-CHA | 22.3 | 20.4 | 82.6 |
| 3 | CuO-ZnO@Cu-MOR | 18.7 | 22.8 | 79.3 |
| | CuO-ZnO/Cu-MOR | 17.1 | 20.3 | 78.5 |
| 4 | CuO-ZnO-ZrO₂@Cu-MOR | 19.3 | 26.5 | 83.7 |
| | CuO-ZnO-ZrO₂/Cu-MOR | 17.8 | 23.6 | 80.5 |
| 5 | CuO-ZnO-Al₂O₃@Cu-MOR | 20.3 | 27.1 | 85.2 |
| | CuO-ZnO-ZrO₂/Cu-MOR | 18.5 | 26.7 | 83.6 |
| 6 | CZAZ/Fe-ZSM-5 | 18.5 | 23.5 | 76.6 |
| 7 | CZAZ/Fe-MOR | 17.4 | 22.7 | 74.1 |
| 8(Compar ative example) | CZAZ/Cu-MOR | 13.5 | 20.5 | 79.2 |
| 9(Compar ative example) | CZAZ | 0 | 26.5 | 90.5 |
| | Cu-MOR | 0 | 0 | 0 |

It can be seen that in the process of production of methanol using a bifunctional catalyst to catalyze methane oxidation coupled with carbon dioxide hydrogenation, when the first catalyst was modified with polydopamine, the conversion rate of CH₄ reaches 28.2%, which is **more than doubled** that of the first catalyst without polydopamine modification (13.5%). In addition, under the same conditions, the bifunctional catalyst with polydopamine modification on the first catalyst has higher methanol selectivity and carbon dioxide selectivity.

The bifunctional catalyst of the present invention is added and used simultaneously in the same container, rather than undergoing a two-step reaction separately in two different containers. Meanwhile, from the comparative Example 9 of the present application, it can also be seen that when the first catalyst and the second catalyst of the bifunctional catalyst of the present application are separately used in the reaction system of catalytic methane oxidation coupled with carbon dioxide hydrogenation to prepare methanol of the present invention, the first catalyst (Cu-MOR) does not have any conversion effect, and the second catalyst can only convert carbon dioxide hydrogenation to prepare methanol and cannot catalyze methane conversion.

The above results indicate that the first catalyst (zeolite molecular sieve) in the bifunctional catalyst of the present application, after modification with polydopamine, can significantly increase the efficiency of methane oxidation and carbon dioxide hydrogenation, as well as the selectivity of methanol, which is of great significance for green and efficient elimination and utilization of greenhouse gases.

All documents referred to in the present invention are incorporated by reference herein as if each document is individually incorporated by reference. Further, it should be understood that upon reading the above teachings of the present invention, various modifications or changes may be made to the present invention by those skilled in the art, and those equivalents also fall within the scope defined by the appended claims of the present application.

## Claims

1. A bifunctional catalyst comprising a first catalyst for catalyzing methane oxidation to prepare methanol and a second catalyst for catalyzing carbon dioxide hydrogenation to prepare methanol; wherein, the first catalyst has a polydopamine modification layer.

2. The bifunctional catalyst according to claim 1, wherein the mass ratio of the first catalyst to the second catalyst is 3:1 to 1:1; preferably 1.5:1 to 1:1; most preferably 1:1.

3. The bifunctional catalyst according to claim 1, wherein the first catalyst is a polydopamine modified metal ion-exchanged zeolite molecular sieve; preferably, the metal ion-exchanged zeolite molecular sieve is selected from the group consisting of: iron ion-exchanged zeolite molecular sieve, copper ion-exchanged zeolite molecular sieve, cobalt ion-exchanged zeolite molecular sieve, nickel ion-exchanged zeolite molecular sieve, or combinations thereof.

4. The bifunctional catalyst according to claim 1, wherein the second catalyst is selected from the group consisting of: metal oxide, metal alloy, noble metal, or combinations thereof.

5. The bifunctional catalyst according to claim 1, wherein the bifunctional catalyst is selected from the group consisting of: CZAZ@Cu-ZSM-5, CZAZ@Cu-MOR, CZAZ@Cu-CHA, CZAZ@Cu-BEA, CZAZ@Cu-FER, CZAZ/Cu-ZSM-5, CZAZ/Cu-MOR, CZAZ/Cu-CHA, CuO-ZnO@Cu-MOR, CuO-ZnO/Cu-MOR, CuO-ZnO-ZrO₂@Cu-MOR, CuO-ZnO-ZrO₂/Cu-MOR, CuO-ZnO-Al₂O₃@Cu-MOR, CuO-ZnO-ZrO₂/Cu-MOR, CZAZ/Fe-ZSM-5, CZAZ/Fe-MOR, or combinations thereof.

6. A method for preparing the bifunctional catalyst according to claim 1, wherein comprising the steps of: preparing the first catalyst and the second catalyst through a wet process or a dry process to obtain the bifunctional catalyst.

7. The method according to claim 6, wherein when preparing the bifunctional catalyst using a wet process, the method comprises the following steps:
(i) dissolving and dispersing a first catalyst precursor in C1-C6 alcoholic solvent to obtain a mixture;
(ii) adding the second catalyst to the mixture and reacting at room temperature to obtain the bifunctional catalyst.

8. The method according to claim 6, wherein when preparing the bifunctional catalyst using a dry process, the method comprises the following steps:
mixing the dry first catalyst and second catalyst evenly to obtain the bifunctional catalyst;
wherein, the mass ratio of the first catalyst to the second catalyst is 3:1 to 1:1, preferably 1.5:1 to 1:1, and more preferably 1:1.

9. Use of the bifunctional catalyst, wherein the bifunctional catalyst is used for preparing methanol by catalyzing methane oxidation coupled with carbon dioxide hydrogenation, wherein the bifunctional catalyst comprises a first catalyst for catalyzing methane oxidation to prepare methanol and a second catalyst for catalyzing carbon dioxide hydrogenation to prepare methanol.

10. A method for preparing methanol by catalyzing methane oxidation coupled with carbon dioxide hydrogenation, wherein comprising the following steps:
in the same reactor, in the presence of the bifunctional catalyst according to claim 1, under a condition of 100-400 °C, introducing a reaction inlet gas containing methane, carbon dioxide, and hydrogen to perform the reaction, thereby preparing methanol, wherein, the inlet gas space velocity of the reactor is 3000 to 10000 h⁻¹, and the inlet gas of the reaction contains 5 to 30% by volume of methane, 5 to 30% by volume of carbon dioxide, and 10 to 60% by volume of hydrogen.
